# EUROPÄISCHE PATENTSCHRIFT

(11) **EP 2 721 585 B1**
(45) Veröffentlichungstag und Bekanntmachung des Hinweises auf die Patenterteilung: **19.12.2018**
(21) Anmeldenummer: 12732804.5
(22) Anmeldetag: 13.06.2012
(51) Int. Cl.: G06T 19/00

(54) **VERFAHREN UND VORRICHTUNG ZUM ERMITTELN UND WIEDERGEBEN VIRTUELLER ORTSBEZOGENER INFORMATIONEN FÜR EINEN RAUMBEREICH**
METHOD AND DEVICE FOR DETERMINING AND REPRODUCING VIRTUAL, LOCATION-BASED INFORMATION FOR A REGION OF SPACE
PROCÉDÉ ET DISPOSITIF PERMETTANT DE DÉTERMINER ET DE REPRODUIRE DES INFORMATIONS LOCALES VIRTUELLES CONCERNANT UNE ZONE SPATIALE

(30) Priorität: 15.06.2011 DE 102011104524
(43) Veröffentlichungstag der Anmeldung: 23.04.2014
(73) Patentinhaber: IFAKT GmbH, 70563 Stuttgart (DE)
(72) Erfinder: SCHUBERT, Lars, 70565 Stuttgart (DE); GRUBE, Mathias, 70771 Leinfelden-Echterdingen (DE); HEIM, Armand, 72770 Reutlingen (DE); TUERCK, Stefan, 88441 Mittelbiberach (DE)
(74) Vertreter: Witte, Weller & Partner Patentanwälte mbB
(86) Internationale Anmeldenummer: PCT/EP2012/061195
(87) Internationale Veröffentlichungsnummer: WO 2012/171955

(56) Entgegenhaltungen:
- US-A1- 2007 164 988
- HUEI-YUNG LIN ET AL: "Augmented Reality with Human Body Interaction Based on Monocular 3D Pose Estimation", ADVANCED CONCEPTS FOR INTELLIGENT VISION SYSTEMS, SPRINGER BERLIN HEIDELBERG, BERLIN, HEIDELBERG, Bd. 6474, 13. Dezember 2010 (2010-12-13), Seiten 321-331, XP019159135, ISBN: 978-3-642-17687-6
- CHUNG-CHING CHANG ET AL: "Pose and Gaze Estimation in Multi-camera Networks for Non-restrictive HCI", HUMANÂ COMPUTER INTERACTION; [LECTURE NOTES IN COMPUTER SCIENCE], SPRINGER BERLIN HEIDELBERG, BERLIN, HEIDELBERG, Bd. 4796, 20. Oktober 2007 (2007-10-20), Seiten 128-137, XP019081888, ISBN: 978-3-540-75772-6

## Beschreibung

Die vorliegende Erfindung betrifft ein Verfahren und eine entsprechende Vorrichtung zum Ermitteln und Wiedergeben virtueller ortsbezogener Informationen für einen Raumbereich. Die vorliegende Erfindung betrifft ferner eine Steuer- und Auswerteeinheit für eine Vorrichtung zum Ermitteln und Wiedergeben virtueller ortsbezogener Informationen für einen Raumbereich. Weiter betrifft die Erfindung ein Verfahren zum Betreiben einer solchen Steuer- und Auswerteeinheit. Schließlich betrifft die vorliegende Erfindung ein Computerprogramm mit Programmcodemitteln zum Durchführen eines Verfahrens der vorgenannten Art.

Ein Verfahren und eine Vorrichtung zum Ermitteln und Wiedergeben virtueller ortsbezogener Informationen für einen Raumbereich ist beispielsweise aus US 2007/0164988 A1 bekannt. Dort ist vorgesehen, dass eine mobile Vorrichtung eine Kamera aufweist, die einen Umgebungsbereich erfassen kann. Eine optische Ausrichtung der Kamera relativ zu der Vorrichtung wird in Abhängigkeit eines Sichtwinkels eines Benutzers geändert. Für eine Bestimmung des Sichtwinkels wird eine Ausrichtung der Pupillen des Benutzers relativ zu der Vorrichtung gemessen und ausgewertet. Anschließend wird eine Position der Vorrichtung ermittelt und zusammen mit der optischen Ausrichtung der Kamera ausgewertet, um die virtuellen ortsbezogenen Informationen ermitteln zu können. Schließlich werden die virtuellen ortsbezogenen Informationen von der Vorrichtung wiedergegeben.
In dem Artikel Huei-Yung Lin et al.: "Augmented Reality with Human Body Interaction Based on Monocular 3D Pose Estimation", Advanced Concepts for Intelligent Vision Systems, Springer, Band 6474, 13. Dezember 2010, Seiten 321-331 ist ein Interface für erweiterte Realität (augmented reality) beschrieben, das ohne Marker und nur durch Körperinteraktion funktioniert. Das Interface umfasst die Erfassung der 3D-Bewegung des menschlichen Körpers und die Verarbeitung von menschlichen 3D-Posen für Anwendungen bei erweiterter Realität. Eine Monokular-Kamera wird dazu benutzt, die Bilder der Nutzerbewegung für eine 3D-Posenschätzung zu erfassen. Zunächst wird ein grafisches 3D-Menschenmodell konstruiert. Dessen Projektion auf eine virtuelle Bildebene wird dann dazu benutzt, die von der Bildsequenz erhaltenen Silhouetten anzugleichen. Durch iteratives Anpassen der 3D-Pose des grafischen 3D-Modells an die physikalischen und anatomischen Beschränkungen der menschlichen Bewegung können die menschliche Pose und die zugeordneten SD-Bewegungsparameter eindeutig identifiziert werden. Die erhaltene Information über die 3D-Pose wird dann in ein Untersystem, das die Realität verarbeitete, übertragen und dazu benutzt, die Marker-freie Interaktion in der erweiterten Realität zu erhalten. Experimentelle Ergebnisse werden zudem unter Benutzung eines am Kopf montierten Displays vorgestellt.

Der vorliegenden Erfindung liegt die Aufgabe zugrunde, ein Verfahren sowie eine Vorrichtung bereitzustellen, die im Vergleich zu herkömmlichen Verfahren und Vorrichtungen eine robuste, schnelle und genaue Ermittlung und Wiedergabe von virtuellen ortsbezogene Informationen für einen Raumbereich ermöglichen.

Gemäß einem Aspekt der vorliegenden Erfindung wird diese Aufgabe durch ein Verfahren gemäß Anspruch 1 gelöst.

Gemäß einem weiteren Aspekt der vorliegenden Erfindung wird diese Aufgabe durch eine Vorrichtung gemäß Anspruch 10 gelöst.

Gemäß einem weiteren Aspekt der vorliegenden Erfindung wird eine Steuer- und Auswerteeinheit gemäß Anspruch 11 zur Verwendung in einer Vorrichtung der eingangs genannten Art bereitgestellt.

Gemäß einem weiteren Aspekt der vorliegenden Erfindung wird ein Verfahren zum Betreiben einer Steuer- und Auswerteeinheit gemäß Anspruch 12 bereitgestellt.

Gemäß einem weiteren Aspekt der vorliegenden Erfindung wird ein Computerprogramm bereitgestellt, mit Programmcodemitteln zum Durchführen der Schritte eines Verfahrens der vorstehend genannten Art, wenn das Computerprogramm auf einem Computer ausgeführt wird.

Der Erfindung liegt die Erkenntnis zugrunde, dass die Sichtrichtung des Betrachters in Abhängigkeit seiner Körperhaltung und seiner Raumposition ermittelt werden kann. Ist die Sichtrichtung des Betrachters relativ zu dem Raumbereich bekannt, so können virtuelle ortsbezogene Informationen ermittelt werden, die einem Teil des Raumbereichs zugeordnet sind, auf den die Sichtrichtung weist. Versuche der Anmelderin haben gezeigt, dass dieser Ansatz in vorteilhafter Weise sehr robust gegenüber Bewegungen des Betrachters ist und zu sehr genauen Ergebnissen beim Ermitteln und Wiedergeben der virtuellen ortsbezogenen Informationen führt. Besonders vorteilhaft ist es, dass die Erfindung keine speziellen Markierungen im Raumbereich oder an dem Betrachter benötigt. Insbesondere erfolgt das Ermitteln der Körperhaltung ohne spezielle Markierungen am Körper des Betrachters.

Der Betrachter wird von einer Sensorvorrichtung erfasst, die Messdaten über den Betrachter ermittelt. Hierbei kann der gesamte Betrachter von der Sensorvorrichtung erfasst werden. Alternativ ist es denkbar, dass nur ein Teil des Körpers des Betrachters erfasst wird, aus dem die Körperhaltung ermittelt werden kann.

Die Sensorvorrichtung weist vorzugsweise mindestens einen bildgebenden Sensor auf, wie beispielsweise eine Digitalkamera, um den mindestens einen Teil des Körpers des Betrachters zu erfassen. Bei Verwendung von bildgebenden Sensoren werden die entsprechenden Messdaten in Form von Bilddaten ermittelt.

Anhand der Messdaten wird die Körperhaltung des Betrachters ermittelt. Unter der Körperhaltung wird eine räumliche Ausrichtung von mindestens einem Körperteil des Betrachters, insbesondere von Armen, Torso und/oder Hals, oder einem Teil des Körperteils verstanden. Bevorzugt ist es, wenn die Körperhaltung in Abhängigkeit von mehreren Körperteilen und der Ausrichtung der Körperteile zueinander ermittelt wird.

Die Körperhaltung ermöglicht einen Rückschluss darauf, auf welchen Teil des Raumbereichs der Betrachter blickt. In einer bevorzugten Ausgestaltung wird die Sichtrichtung unter der Annahme ermittelt, dass der Betrachter die Anzeigevorrichtung in mindestens einer seiner Hände hält. Unter zusätzlicher Annahme, dass der Betrachter auf die Anzeigevorrichtung blickt, kann die Sichtrichtung in Abhängigkeit der räumlichen Ausrichtung des Kopfs und der Arme direkt ermittelt werden. In einer alternativen Ausgestaltung wird die Sichtrichtung unter der Annahme ermittelt, dass der Betrachter über die Anzeigevorrichtung blickt. Dann kann die Sichtrichtung in Abhängigkeit der räumlichen Ausrichtung des Kopfs, der Arme und einer entsprechenden Anpassung ermittelt werden, die die Anzeigevorrichtung berücksichtigt. In einer besonders bevorzugten Ausgestaltung wird die Ausrichtung des Kopfs und der Arme des Betrachters zueinander ausgewertet, um automatisch zu ermitteln, ob der Betrachter auf die Anzeigevorrichtung blickt oder ob er an der Anzeigevorrichtung vorbei blickt. Die Ermittlung der Sichtrichtung kann dann automatisch an den jeweiligen Fall angepasst werden.

Um die Sichtrichtung relativ zu dem Raumbereich ermitteln zu können, wird zusätzlich die Raumposition des Betrachters relativ zu dem Raumbereich bestimmt. Die Raumposition kann als Raumpunkt mit Raumkoordinaten in dem Raumbereich repräsentiert werden, wobei die Position des Raumpunkts relativ zum Körper des Betrachters bekannt ist.

Vorzugsweise ist es vorgesehen, dass eine Sensorposition sowie eine Sensorausrichtung der Sensorvorrichtung innerhalb des Raumbereichs bekannt sind. Die Sensorposition und/oder die Sensorausrichtung können beispielsweise durch Kalibrieren der Sensorvorrichtung ermittelt werden. Sind Sensorposition und Sensorausrichtung bekannt, ist es ausreichend, wenn eine relative Position des Betrachters zu der Sensorvorrichtung bestimmt wird, da diese relative Position anschließend in Abhängigkeit der Sensorposition und Sensorausrichtung in die Raumposition des Betrachters relativ zu dem Raumbereich transformiert werden kann. Die Bestimmung der Raumposition relativ zu dem Raumbereich ist notwendig, um die virtuellen ortsbezogenen Informationen in Abhängigkeit der Sichtrichtung korrekt ermitteln zu können.

Die virtuellen ortsbezogenen Informationen sind vorzugsweise Informationen, die bestimmten Teilen des Raumbereichs zugeordnet sind. Die Zuordnung erfolgt dann innerhalb eines virtuellen Koordinatensystems, in dem die virtuellen ortsbezogenen Informationen organisiert sind. Die Ausrichtung und Position des virtuellen Koordinatensystems relativ zu dem Raumbereich sind vorzugsweise bekannt. Ist die Sichtrichtung relativ zu dem Raumbereich ermittelt, kann diese in das virtuelle Koordinatensystem transformiert werden, um die ortsbezogenen virtuellen Daten zu ermitteln.

Weiter ist es vorzugsweise vorgesehen, dass die virtuellen ortsbezogenen Informationen in einer Datenbank gespeichert sind. Die Datenbank kann beispielsweise in der Steuer- und Auswerteeinheit, der Anzeigevorrichtung und/oder in einem Netzwerk hinterlegt sein, auf das die Steuer- und Auswerteeinheit oder die Anzeigevorrichtung Zugriff hat. Bei den virtuellen ortsbezogenen Informationen kann es sich beispielsweise um Textinformationen handeln, die einzelnen Abschnitten oder Punkten des Raumbereichs zugeordnet sind. Alternativ oder zusätzlich können sie graphische Informationen sein, die den betrachteten Teil des Raumbereichs virtuell und vorzugsweise mit zusätzlichen Informationen ergänzt darstellen.

Beispielsweise ist es denkbar, dass sich der Betrachter in einem Flugzeugrumpf befindet, welcher sich im Rohbau befindet. In Abhängigkeit seiner Sichtrichtung werden dem Betrachter Informationen darüber wiedergegeben, welche Bauelemente einzusetzen, zu prüfen oder zu ersetzen sind. Zudem ist es denkbar, dass ihm angezeigt wird, welche Arbeitsschritte er für die entsprechende Tätigkeit durchführen muss. Auch eine Kontrolle des korrekten Einbaus von Bauteilen ist damit einfach möglich.

Als weiteres Beispiel ist es denkbar, dass die Vorrichtung und das Verfahren in einem medizinischen Bereich eingesetzt werden. Einem Arzt könnten beispielsweise in Abhängigkeit seiner Sichtrichtung auf einen Patienten eine Auswahl geeigneter CT-Bilder angezeigt werden, die den Körperbereich des Patienten betreffen, auf den der Arzt blickt.

Als weiteres Beispiel ist es denkbar, dass ein Betrachter innerhalb eines Hauses auf einen Wandbereich blickt und ihm als virtuelle ortsbezogene Informationen Teile eines Plans des Hauses angezeigt werden. Dieser kann ihn dann über Leitungsverläufe informieren oder gestalterische Variationen des Wandbereichs anzeigen.

Als weiteres Beispiel ist es denkbar, dass sich der Betrachter in einem Waggon befindet. Der Waggon kann hierbei teilweise oder vollständig hergestellt sein. In Abhängigkeit seiner Sichtrichtung werden dem Betrachter virtuelle ortsbezogene Informationen darüber wiedergegeben, welche Bauelemente einzusetzen, zu prüfen oder zu ersetzen sind. Insbesondere können ihm hierbei virtuelle ortsbezogene Informationen darüber gegeben werden, wie elektrische Leitungen und/oder Druckleitungen verlaufen oder verlaufen sollen. Somit ist es denkbar, dass ihm auch angezeigt wird, welche Arbeitsschritte er für entsprechende Tätigkeiten, wie Einbau- oder Wartungstätigkeiten, durchführen muss. Auch eine Kontrolle des korrekten Einbaus von Bauteilen, insbesondere der elektrischen Leitungen und der Druckleitungen, ist damit sehr einfach möglich.

Als weiteres Beispiel ist es denkbar, dass sich der Betrachter in einem teilweise oder vollständig hergestellten Schiffsrumpf befindet. In Abhängigkeit seiner Sichtrichtung werden dem Betrachter virtuelle ortsbezogene Informationen darüber wiedergegeben, welche Bauelemente einzusetzen, zu prüfen oder zu ersetzen sind. Hierbei können insbesondere elektrische Leitungen, Rohrleitungen und Verkleidungsteile berücksichtigt werden. Es ist zudem auch denkbar, dass ganze Baugruppen, die in den Schiffsrumpf integriert werden sollen, angezeigt werden, wie beispielsweise eine Brücke oder ein Schiffsmotor. Ferner ist es denkbar, dass dem Betrachter virtuelle ortsbezogene Informationen über sicherheitsrelevante Stellen, wie beispielsweise stark belastete Schweißnähte, angezeigt werden. Der Betrachter kann dann anhand dieser Informationen die entsprechenden Stellen überprüfen und gegebenenfalls mit Referenzdaten vergleichen. Zudem ist es denkbar, dass ihm angezeigt wird, welche Arbeitsschritte er für die entsprechende Tätigkeit durchführen muss. Auch hier ist eine Kontrolle des korrekten Einbaus von Bauteilen einfach möglich. Zudem ist es denkbar, dass dem Betrachter virtuelle ortsbezogene Informationen über Passagierkabinen gegeben werden, wie beispielsweise Informationen über Innenausbauten der entsprechenden Passagierkabinen und Informationen über bestehende Leitungen hinter Wandverkleidungen für Wartungsarbeiten.

Als weiteres Beispiel ist es denkbar, dass die Vorrichtung und das Verfahren im Straßenbau eingesetzt werden. Der Betrachter kann dann beispielsweise in Abhängigkeit seiner Sichtrichtung auf eine Straße virtuelle ortsbezogene Informationen darüber erhalten, wie die Beschaffenheit unterhalb der Straße ist. Insbesondere können ihm virtuelle ortsbezogene Informationen über Leitungen, wie Rohrleitungen, Gasleitungen und elektrische Leitungen, gegeben werden. In anderen Worten, der Betrachter kann virtuell durch die Straße hindurch in den Boden schauen. Ferner ist es denkbar, dass dem Betrachter als virtuelle ortsbezogene Informationen Ausschnitte einer entsprechenden Bauzeichnung für einen betrachteten Bereich in Sichtrichtung angezeigt werden. Somit wird der Betrachter für entsprechende Bauarbeiten schnell und einfach informiert. Zudem kann er bereits getätigte Arbeitsschritte mit Referenzinformationen sehr einfach vergleichen.

Als weiteres Beispiel ist denkbar, dass die Vorrichtung und das Verfahren bei einer Inselfertigung mit einer Montageinsel eingesetzt werden. Inselfertigungen werden beispielsweise bei der Herstellung von Motoren, insbesondere Verbrennungsmotoren, eingesetzt. Der Motor ist dabei zentral angeordnet, wobei ein Monteur um den Motor herumgehen kann, um diesen von unterschiedlichen Seiten her bearbeiten zu können. Hierbei ermöglicht das erfindungsgemäße Verfahren sowie die Vorrichtung, dass dem Monteur in Abhängigkeit seine Position direkt die virtuellen ortsbezogenen Informationen bereitgestellt werden, die er für Arbeitsschritte in dem Bereich benötigt, in dem er sich befindet. Insbesondere bei der Inselfertigung ist es denkbar, dass es sich bei der Anzeigevorrichtung um einen fest installierten Monitor oder einen schwenkbaren Monitor handelt. Dieser kann beispielsweise drehbar oberhalb der Montageinsel angeordnet sein. Alternativ oder zusätzlich ist es denkbar, dass mehrere Monitore gleichzeitig eingesetzt werden, die aus verschiedenen Positionen des Monteurs betrachtet werden können. Dabei ist es denkbar, dass dem Monteur angezeigt wird, welche Arbeitsschritte er für die entsprechenden Tätigkeiten in dem Bereich durchführen muss auf den seine Sichtrichtung deutet. Zudem kann ihm angezeigt werden, welche Bauteile er für diese Tätigkeiten benötigt. Ferner kann ihm angezeigt werden, wie die Teile einzusetzen sind und welche Lage die Teile beim Einsetzen haben sollen. Zudem ist auch eine Kontrolle des korrekten Einbaus der Bauteile sehr einfach möglich.

Die Anzeigevorrichtung ist vorzugsweise derart ausgebildet, dass sie die virtuellen ortsbezogenen Informationen optisch wiedergeben kann. Alternativ oder zusätzlich ist es denkbar, dass die virtuellen ortsbezogenen Informationen akustisch und/oder haptisch wiedergegeben werden. Hierdurch können die bereits genannten Beispiele durch zusätzliche Wiedergabemöglichkeiten erweitert werden. Ferner ist es denkbar, dass akustische und/oder haptische Wiedergaben Personen mit einer Sehbehinderung Informationen über den entsprechenden Teil des Raumbereichs geben können.

Ein weiterer Vorteil der Erfindung ist es, dass die virtuellen ortsbezogenen Informationen unabhängig von einer Position der Anzeigevorrichtung ermittelt werden können. Es werden lediglich die virtuellen ortsbezogenen Informationen ermittelt und wiedergegeben, die der tatsächlichen Sichtrichtung des Betrachters entspricht. Hierdurch wird eine Steuerung zur Wiedergabe der virtuellen ortsbezogenen Informationen vereinfacht, da diese implizit durch Bewegungen des Betrachters ausgewählt werden und entsprechend angepasst werden können.

Weiter ist es denkbar, dass die Sichtrichtung durch manuelle Eingabe des Benutzers gesperrt werden kann. Hierbei ist es vorgesehen, dass der Betrachter die Sichtrichtung und damit die aktuell angezeigten virtuellen ortsbezogenen Informationen fixiert. Somit kann sich der Betrachter frei im Raumbereich bewegen, ohne dass sich für ihn relevante Informationen auf der Anzeigevorrichtung verändern.

Alternativ oder zusätzlich ist es denkbar, dass der Betrachter die Sichtrichtung manuell manipulieren kann, beispielsweise durch ein Eingabegerät wie eine Maus, ein Trackball oder ein Touchscreen. Somit kann der Betrachter zusätzlich unabhängig von seiner Körperhaltung relevante Informationen gezielt aufrufen, wenn er diese benötigt.

Bevorzugt ist es, wenn die Steuer- und Auswerteeinheit als Computer, beispielsweise als PC, ausgebildet ist. In weiteren bevorzugten Ausgestaltungen kann die Steuer- und Auswerteeinheit aus einer Vielzahl von Berechnungseinheiten, wie beispielsweise Mikroprozessoren, bestehen, die miteinander kommunizieren. Weiter können die Sensorvorrichtung und/oder die Anzeigevorrichtung eigene Berechnungseinheiten aufweisen. Die Steuer- und Auswerteeinheit kann somit als eigenständige Einheit ausgebildet sein. Alternativ kann sie als Zusammenschluss mehrerer Berechnungseinheiten ausgebildet sein, wobei diese Berechnungseinheiten bevorzugt in unterschiedlichen Baugruppen der erfindungsgemäßen Vorrichtung angeordnet sind. Als weitere Alternative kann die Steuer- und Auswerteeinheit auch vollständig in der Sensorvorrichtung und/oder der Anzeigevorrichtung integriert sein.

In einer Ausgestaltung der Erfindung werden als Messdaten dreidimensionale Messdaten ermittelt. In dieser Ausgestaltung wird dann die Körperhaltung des Betrachters in Abhängigkeit von dreidimensionalen Messdaten ermittelt. Die Messdaten weisen zusätzlich zu Höhen- und Breiteninformationen auch Tiefeninformationen auf. Hierdurch kann die Körperhaltung des Betrachters besonders genau ermittelt werden. Der Einsatz dreidimensionaler Messdaten ermöglicht zudem ein noch genaueres Bestimmen der Raumposition des Betrachters.

Zum Ermitteln der dreidimensionalen Messdaten kann die Sensorvorrichtung als Sensor eine 3D-Kamera aufweisen, beispielsweise eine Stereokamera. In weiteren bevorzugten Ausgestaltungen weist die Sensorvorrichtung als Sensor eine digitale (2D-)Kamera, einen Infrarot-Tiefensensor, einen Laser-Tiefensensor, eine Flug-Distanz-Kamera und/oder einen Ultraschallsensor auf.

Erfindungsgemäß wird die Sensorvorrichtung betrachterunabhängig und/oder ortsfest in dem Raumbereich angeordnet. Bei betrachterunabhängier Anordnung ist die Sensorvorrichtung von dem Betrachter entfernt und physisch unabhängig angeordnet. Ein Vorteil hierbei ist es, dass aufgrund der betrachterunabhängigen Anordnung die Sensorposition sowie die Sensorausrichtung sehr exakt bestimmbar sind. Vorteilhaft ergibt sich daraus eine besonders genaue Erfassung der Sichtrichtung des Betrachters.

Zusätzlich ist es denkbar, dass sich die Sensorvorrichtung automatisch oder durch gezielte Steuerung des Betrachters an die Raumposition des Betrachters anpasst. Hierdurch kann die Sensorvorrichtung dem Betrachter folgen, um einen größeren Raumbereich abzudecken.

Bei ortsfester Anordnung ist die Sensorvorrichtung relativ zu dem Raumbereich fest angeordnet. Hieraus ergibt sich eine gleichbleibende Sensorposition, wodurch das Bestimmen der Raumposition vereinfacht und beschleunigt wird. Zudem ergibt sich der Vorteil, dass die Sensorvorrichtung beispielsweise vor einer Benutzung nur einmalig eingemessen und kalibriert werden muss, so dass hochgenaue Messdaten ermittelt werden können. Ferner ergibt sich der Vorteil, dass die Sensorvorrichtung sehr genau auf Störeinflüsse, wie Licht-und Schattenverhältnisse, in dem erfassten Teil des Raumbereichs kalibriert werden kann. Somit kann die Genauigkeit durch Berücksichtigen der Störeinflüsse des Raumbereichs auf die Sensorvorrichtung zusätzlich verbessert werden.

In einer weiteren Ausgestaltung der Erfindung ermittelt die Sensorvorrichtung automatisch eine Sensorposition relativ zu dem Raumbereich. In dieser Ausgestaltung wird die Sensorposition beispielsweise direkt in Abhängigkeit der Messdaten der Sensorvorrichtung ermittelt. Dies kann beispielsweise im Zuge eines Kalibriervorgangs erfolgen.

Bevorzugt erfasst die Sensorvorrichtung den Raumbereich innerhalb eines Sensorbereichs und gleicht die entsprechenden Messdaten mit virtuellen Daten über die Struktur des Raumbereichs ab. Ein Vorteil hierbei ist es, dass das Kalibrieren der Sensorvorrichtung besonders schnell und genau erfolgen kann. Zudem ist es denkbar, dass die Sensorposition wiederkehrend ermittelt wird, so dass dauerhaft eine sehr hohe Genauigkeit beim Ermitteln der Sichtrichtung gewährleistet ist.

Alternativ oder zusätzlich ist denkbar, dass die Sensorvorrichtung die Sensorposition mittels eines Positionssensors ermittelt. Vorzugsweise weist die Sensorvorrichtung den Positionssensor auf. Der Positionssensor kann beispielsweise als GPS-Sensor ausgebildet sein. Der GPS-Sensor hat den Vorteil, dass auch dann die Sensorposition sehr genau ermittelt werden kann, wenn der Raumbereich für die Sensorvorrichtung zunächst unbekannt ist. Besonders bevorzugt ist es, wenn der GPS-Sensor ein Differential-GPS-Sensor ist. Alternativ oder zusätzlich ist es denkbar, dass Funkpositionssysteme eingesetzt werden. Derartige Funkpositionssysteme ermitteln die Sensorposition anhand von Funkpeilung und/oder Triangulation. Diese haben den Vorteil, dass sie in geschlossenen Räumen eingesetzt werden können und die Sensorposition hochgenau ermitteln. Derartige Funkpeilungssysteme können beispielsweise mittels WLAN, Bluetooth und/oder UMTS realisiert werden. Als weitere Alternative ist es denkbar, dass Lasersensoren zur Bestimmung der Sensorposition eingesetzt werden.

In einer weiteren Ausgestaltung ermittelt die Sensorvorrichtung automatisch eine Sensorausrichtung relativ zu dem Raumbereich. In dieser Ausgestaltung wird die Sensorausrichtung direkt anhand der Messdaten der Sensorvorrichtung ermittelt. Dies kann beispielsweise im Zuge eines Kalibriervorgangs erfolgen.

Bevorzugt erfasst die Sensorvorrichtung den Raumbereich innerhalb seines Sensorbereichs und gleicht die entsprechenden Messdaten mit virtuellen Daten über die Struktur des Raumbereichs ab. Ein Vorteil hierbei ist es, dass das Kalibrieren der Sensorvorrichtung besonders schnell und genau erfolgt. Zudem ist es denkbar, dass die Sensorausrichtung wiederkehrend ermittelt wird, so dass dauerhaft eine sehr hohe Genauigkeit beim Ermitteln der Sichtrichtung gewährleistet ist.

Alternativ oder zusätzlich ist denkbar, dass die Sensorvorrichtung die Sensorlage durch einen Lagesensor ermittelt. Der Lagesensor kann beispielsweise als GPS-Sensor, Beschleunigungssensor und/oder als Kompass-Chip ausgebildet sein. Diese Lagesensoren haben den Vorteil, dass auch dann die Sensorlage sehr genau ermittelt werden kann, wenn der Raumbereich für den Sensor zunächst unbekannt ist. Besonders bevorzugt ist es, wenn der GPS-Sensor ein Differential-GPS-Sensor ist. Alternativ oder zusätzlich ist es denkbar, dass Funkpositionssysteme eingesetzt werden. Derartige Funkpositionssysteme ermitteln die Sensorlage anhand von Funkpeilung und/oder Triangulation. Diese haben den Vorteil, dass sie in geschlossenen Räumen eingesetzt werden können und eine hochgenaue Sensorlage ermitteln. Derartige Funkpeilungssysteme können beispielsweise mittels W-LAN, Bluetooth und/oder UMTS realisiert werden. Als weitere Alternative ist es denkbar, dass Lasersensoren zur Bestimmung der Sensorlage eingesetzt werden.

In einer weiteren Ausgestaltung wird eine Vielzahl von Sensorvorrichtungen eingesetzt. In dieser Ausgestaltung wird die Vielzahl von Sensorvorrichtungen verwendet, die gemeinsam die Messdaten ermitteln.

Bevorzugt ist es vorgesehen, dass verschiedene Sensorvorrichtungen unterschiedliche oder zumindest teilweise unterschiedliche Teile des Raumbereichs erfassen. Hierdurch kann der Sensorbereich vergrößert werden, in dem der Betrachter erfasst wird.

Alternativ oder zusätzlich ist es denkbar, dass mehrere Sensorvorrichtungen gemeinsam einen Teil des Raumbereichs erfassen. Ein Vorteil hierbei ist es, dass der Betrachter aus unterschiedlichen Richtungen erfasst wird und somit die Messdaten der Sensorvorrichtungen miteinander verglichen werden können. Somit kann eine höhere Genauigkeit und Robustheit beim Bestimmen der Raumposition und Ermitteln der Körperhaltung des Betrachters erreicht werden.

In einer weiteren Ausgestaltung der Erfindung werden als der mindestens eine Teil des Körpers des Betrachters mindestens ein Armbereich des Betrachters verwendet. In dieser Ausgestaltung wird nur ein Teil des Körpers des Betrachters zur Ermittlung der Sichtrichtung berücksichtigt. Versuche der Anmelderin haben gezeigt, dass Messdaten von dem Körper des Betrachters aus dem Bereich der Arme ausreichend sind, um die Sichtrichtung sehr genau ermitteln zu können. Ein Vorteil hierbei ist es, dass die zu verarbeitenden Messdaten verringert werden und somit ein Ablauf des Verfahrens unter Beibehaltung der Messgenauigkeit beschleunigt wird.

In dieser Ausgestaltung ist es insbesondere denkbar, dass eine räumliche Lage sowie eine räumliche Position des Unterarms und des Oberarms des Betrachters zur Bildung der Sichtrichtung erfasst werden. Die Sichtrichtung kann dann beispielsweise unter der Annahme ermittelt werden, dass sie sich entlang des Unterarms erstreckt. Der Unterarm kann somit von dem Betrachter ähnlich wie ein Zeigestock verwendet werden, um der Vorrichtung die Sichtrichtung vorzugeben. In dieser Ausgestaltung ist es besonders bevorzugt, dass lediglich ein Arm des Betrachters berücksichtigt wird. Versuche der Anmelderin haben gezeigt, dass die Berücksichtigung lediglich eines Arms des Betrachters generell sehr robuste und genaue Ergebnisse liefen kann.

Zudem kann es vorgesehen sein, dass ein Schulterbereich des Betrachters zusätzlich berücksichtigt wird. In Abhängigkeit des Schulterbereichs kann eine räumliche Ausrichtung des gesamten Arms noch genauer ermittelt werden.

Zudem kann es vorgesehen sein, dass ein Halsansatz des Betrachters zusätzlich berücksichtigt wird. In Abhängigkeit des Halsansatzes kann eine räumliche Ausrichtung des Kopfs ermittelt werden. Ein Vorteil hierbei ist es, dass die Genauigkeit weiter erhöht wird. Diese Ausgestaltung ist insbesondere dann vorteilhaft, wenn als Sichtrichtung eine Richtung von dem Kopf des Betrachters zu einem Bereich der Hände des Betrachters angenommen wird.

In einer weiteren Ausgestaltung der Erfindung wird als der mindestens eine Teil des Körpers des Betrachters mindestens ein Brustbereich des Betrachters verwendet. In dieser Ausgestaltung wird ebenfalls nur ein Teil des Körpers des Betrachters zur Ermittlung der Sichtrichtung berücksichtigt. Versuche der Anmelderin haben gezeigt, dass Informationen über die Körperhaltung des Betrachters im Bereich der Brust bereits ausreichen, um die Sichtrichtung ermitteln zu können. Ein Vorteil hierbei ist es, dass die zu verarbeitenden Messdaten besonders stark verringert werden und somit ein Ablauf des Verfahrens besonders stark beschleunigt wird.

Diese Ausgestaltung ist insbesondere dann vorteilhaft, wenn als Sichtrichtung eine Richtung angenommen wird, die sich orthogonal aus dem Brustbereich des Betrachters erstreckt. Hierbei wird der Brustbereich vorzugsweise vereinfacht als Fläche betrachtet, wobei die Sichtrichtung dann die Normale bezüglich dieser Fläche ist.

Erfindungsgemäß wird ein Skelettmodell in Abhängigkeit der Messdaten gebildet und die Körperhaltung des Betrachters wird mittels des Skelettmodels ermittelt. Dabei wird der mindestens eine Teil des Körpers des Betrachters zunächst als Skelettmodell approximiert. Dabei ergibt sich typischerweise eine stark vereinfachte Repräsentation des Betrachters ähnlich einem "Strichmännchen". Das Skelettmodell stellt Körperteile des Betrachters vereinfacht als Geraden dar. Zusätzlich zu den vereinfachten Körperteilen weist das Skelettmodell vorzugsweise Gelenkpunkte auf, die die einzelnen Körperteile schwenkbar verbindet. Weiter weist das Skelettmodell für jeden Gelenkpunkt einen Winkel zwischen den jeweiligen Körperteilen auf, die die Körperhaltung eindeutig definieren. In Abhängigkeit dieser vereinfachten Körperteile und Gelenkpunkte können die Körperhaltung und zukünftige Bewegungsmöglichkeiten des Betrachters in vorteilhafter Weise sehr einfach und mit geringem Rechenaufwand ermittelt und analysiert werden.

Das Skelettmodell ist abhängig von dem Teil des Körpers des Betrachters, der von der Sensorvorrichtung erfasst wird. In anderen Worten, wird nur ein Teil des Körpers des Betrachters berücksichtigt, so wird auch nur aus diesem Teil das Skelettmodell gebildet. Ein Vorteil hierbei ist eine weitere Verringerung notwendiger Daten und damit eine Verringerung des Rechenaufwandes.

Bevorzugt ist weiter, dass die Raumposition einen Raumpunkt innerhalb des Skelettmodells darstellt. Dieser Raumpunkt kann dann als Fixpunkt innerhalb des Skelettmodells angeordnet sein. Somit kann die Sichtrichtung sehr einfach in Abhängigkeit der Körperhaltung zu diesem Fixpunkt ermittelt und anschließend in ein Koordinatensystem des Raumbereichs transformiert werden.

In einer weiteren Ausgestaltung der Erfindung wird eine Bewegung der Sichtrichtung erfasst und die Sichtrichtung wird in Abhängigkeit der Bewegung ermittelt. In dieser Ausgestaltung der Erfindung wird bei dem Ermitteln der Sichtrichtung die bisherige Bewegung der Sichtrichtung berücksichtigt. Somit werden Veränderungen der Sichtrichtung über die Zeit erfasst und analysiert. Anschließend wird die bisherige Bewegung dazu eingesetzt, die Sichtrichtung noch genauer zu ermitteln. Beispielsweise können zukünftige Sichtrichtungen vorausberechnet werden. Ein Vorteil hierbei ist es, dass entsprechende virtuelle ortsbezogene Informationen vorgespeichert werden können, wodurch das Verfahren insgesamt beschleunigt wird.

Zudem ist es denkbar, dass die Bewegung gefiltert wird, sodass eine Bewegungsglättung, wie beispielsweise eine Bewegungsfilterung mit einem Tiefpassfilter, bei Ermittlung der Sichtrichtung verwendet werden kann. Ein Vorteil hierbei ist es, dass die Sichtrichtung noch genauer ermittelt wird und Störeinflüsse, wie beispielsweise Sensorrauschen, verringert werden können. Folglich ergibt sich daraus eine besonders stabile, flüssige und störungsfreie Wiedergabe der tatsächlich gewünschten virtuellen ortsbezogenen Informationen. Zudem ergibt sich der Vorteil, dass die ermittelten virtuellen ortsbezogenen Informationen besonders genau mit der realen Sichtrichtung des Betrachters übereinstimmen, da Störeinflüsse minimiert werden.

In einer weiteren Ausgestaltung der Erfindung werden als virtuelle ortsbezogene Informationen CAD-Daten verwendet. Die virtuellen ortsbezogenen Informationen basieren zumindest zum Teil auf CAD-Daten. Ein Vorteil hierbei ist es, dass CAD-Daten typischerweise bei industriellen Anwendungen bereits verfügbar sind. Zudem liegen diese CAD-Daten typischerweise als dreidimensionale Modelle vor.

Besonders bevorzugt ist es, wenn die CAD-Daten den Raumbereich selbst mindestens teilweise virtuell abbilden. Dadurch wird ermöglicht, dass die Sichtrichtung in ein virtuelles Koordinatensystem der CAD-Daten transformiert werden kann. In Abhängigkeit der Sichtrichtung kann dann ein betrachteter Teil des Raumbereichs bestimmt werden. Anschließend können so die jeweiligen virtuellen ortsbezogenen Informationen direkt ermittelt werden. Hierdurch wird eine besonders einfache Zuordnung der Sichtrichtung zu den entsprechenden virtuellen ortsbezogenen Informationen erreicht. Weiter ergibt sich der Vorteil, dass bereits bestehende Systeme sehr einfach und wirtschaftlich zusammen mit der Erfindung verwendet werden können.

In einer weiteren Ausgestaltung der Erfindung wird als Anzeigevorrichtung ein Tablet-Computer verwendet. In dieser Ausgestaltung ist die Anzeigevorrichtung als mobile Einheit in Form eines Tablet-Computers ausgebildet. Unter dem Begriff Tablet-Computer werden insbesondere mobile Geräte verstanden, die zumindest einen Bildschirm sowie einen Grafikprozessor zum Wiedergeben der virtuellen ortsbezogenen Informationen aufweisen, wie beispielsweise ein Tablet-PC oder ein Mobiltelefon.

Alternativ oder zusätzlich ist es denkbar, dass die Anzeigevorrichtung als Headup-Display ausgebildet ist. Das Headup-Display kann beispielsweise als Datenbrille ausgestaltet sein, die von dem Betrachter getragen wird. Dabei weist die Datenbrille mindestens ein optisches Wiedergabemittel auf, welches die virtuellen bezogenen Informationen wiedergeben kann.

In besonders bevorzugten Ausgestaltungen weist die Anzeigevorrichtung eine drahtlose Sende- und Empfangsvorrichtung auf, wie beispielsweise Bluetooth und/oder WLAN. Die Anzeigevorrichtung kann so beispielsweise Daten über die Sichtrichtung und/oder die virtuellen ortsbezogenen Informationen empfangen. Ein Vorteil hierbei ist es, dass der Betrachter in seiner Bewegungsfreiheit nicht eingeschränkt ist. Ein weiterer Vorteil ist es, dass derartige mobile Anzeigevorrichtungen sehr einfach und wirtschaftlich für industrielle Zwecke zur Verfügung stehen. Somit sind eine wirtschaftliche Herstellung der Vorrichtung und eine wirtschaftliche Durchführung des Verfahrens möglich.

In einer weiteren bevorzugten Ausgestaltung weist die Anzeigevorrichtung einen Lagesensor auf. Der Lagesensor kann beispielsweise als GPS-Sensor, Kompass-Chip und/oder Beschleunigungssensor ausgebildet sein. Der Lagesensor der Anzeigevorrichtung ermittelt eine Raumposition und/oder Raumlage des Anzeigegeräts selber. Diese Raumposition und/oder Raumlage der Anzeigevorrichtung kann der Steuer- und Auswerteeinheit zur Verfügung gestellt werden. Die Steuer- und Auswerteeinheit kann dann anhand dieser zusätzlichen Information die Sichtrichtung überprüfen und/oder diese Information beim Ermitteln der Sichtrichtung berücksichtigen. Dies ist insbesondere dann vorteilhaft, wenn die Sichtrichtung ausgehend vom Kopf des Betrachters zu oder über die Anzeigevorrichtung angenommen wird. Als Lagesensor ist auch ein Funkpositionssystem denkbar, das beispielsweise auf WLAN, Bluetooth oder UMTS basiert.

In einer weiteren Ausgestaltung der Erfindung werden zusätzlich reale ortsbezogene Informationen auf der Anzeigevorrichtung wiedergegeben. In dieser Ausgestaltung werden die virtuellen ortsbezogenen Informationen gleichzeitig mit den realen ortsbezogenen Informationen angezeigt. Beispielsweise können die virtuellen ortsbezogenen Informationen die realen ortsbezogenen Informationen überlagern.

Bevorzugt ist es, wenn die Anzeigevorrichtung eine Kamera aufweist, die den Teil des Raumbereichs erfasst, in die die Sichtrichtung zeigt. Zudem ist es denkbar, dass eine Ausrichtung der Kamera für die realen ortsbezogenen Informationen in Abhängigkeit der Sichtrichtung automatisch angepasst wird. Alternativ ist es denkbar, dass eine Kamera im Bereich des Betrachters angeordnet ist, beispielsweise als Helmkamera oder Fingerkamera, um die realen ortsbezogenen Informationen zu erfassen. Die Fingerkamera ist insbesondere dann vorteilhaft, wenn die Sichtrichtung in Abhängigkeit der Armposition ermittelt wird, wobei die Sichtrichtung eine Verlängerung des Unterarms des Betrachters darstellt. Ein Vorteil hierbei ist es, dass dem Betrachter zusätzliche reale Informationen bereitgestellt werden, die die virtuellen ortsbezogenen Informationen direkt in einen Zusammenhang mit dem realen Raumbereich bringen.

Die Erfindung wird nachfolgend anhand von Zeichnungen unter Bezugnahme auf die Erfindung nicht einschränkende Ausführungsbeispiele näher erläutert. Es zeigen:
- Fig. 1: einen bevorzugten Anwendungsort der Erfindung,
- Fig. 2: eine schematische Darstellung eines Ausführungsbeispiels der erfindungsgemäßen Vorrichtung,
- Fig. 3: eine schematische Darstellung unterschiedlicher Koordinatensysteme zur Bestimmung einer Sichtrichtung,
- Fig. 4: ein erstes Skelettmodell eines Betrachters, wobei die Sichtrichtung anhand eines Arm-, Schulter- und Kopfbereichs des Betrachters bestimmt wird,
- Fig. 5: ein zweites Skelettmodell, wobei die Sichtrichtung in Abhängigkeit eines Brustbereichs des Betrachters bestimmt wird, und
- Fig. 6: ein Flussdiagramm eines bevorzugten Ausführungsbeispiels des erfindungsgemäßen Verfahrens.

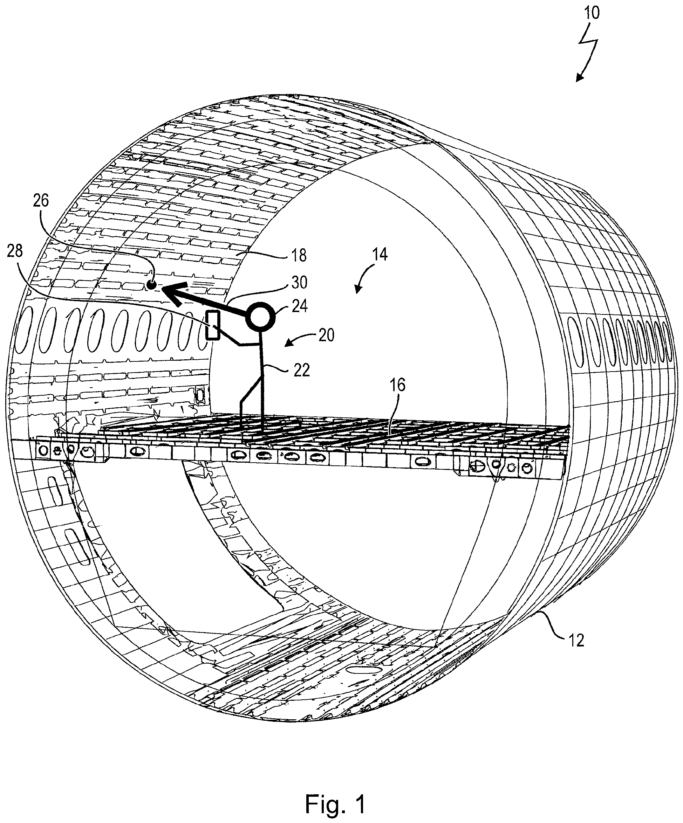

Fig. 1 zeigt einen typischen Einsatzbereich 10 der Erfindung in einer beispielhaften Anwendung. Der Einsatzbereich 10 wird durch einen Flugzeugrumpf 12 gebildet, der hier schematisch und abschnittsweise dargestellt ist. Innerhalb des Flugzeugrumpfes 12 ist ein Raumbereich 14 ausgebildet. Der Raumbereich 14 ist von einem Boden 16 und einer Wand 18 begrenzt. Aufgrund der schematischen Darstellung ist der Raumbereich 14 zu zwei Seiten hin geöffnet. Innerhalb des Raumbereichs 14 befindet sich ein Betrachter 20. Sein Körper 22 und damit auch sein Kopf 24 sind auf einen betrachteten Teil 26 des Raumbereichs 14 an der Wand 18 ausgerichtet. Der Betrachter 20 hält eine Anzeigevorrichtung 28 in seinen Händen. Die Anzeigevorrichtung 28 gibt virtuelle ortsbezogene Informationen wieder, die sich auf den betrachteten Teil 26 des Raumbereichs 14 beziehen.

In dem dargestellten Einsatzbereich 10 können dem Betrachter 20 als virtuelle ortsbezogene Informationen beispielsweise Informationen darüber wiedergegeben werden, welche Herstellungsschritte in dem betrachteten Teil 26 des Raumbereichs 14 zu erfolgen haben. Auch ist es denkbar, dass ihm virtuelle ortsbezogene Informationen darüber gegeben werden, welche Bauteile bereits in dem betrachteten Teil 26 des Raumbereichs 14 vorhanden sein müssen, um somit bereits getane oder noch zu tätigende Herstellungsschritte bewerten zu können. Durch die Erfindung wird es ermöglicht, dass sich der Betrachter 20 in dem Raumbereich 14 frei bewegen kann und ihm gleichzeitig auf der Anzeigevorrichtung 28 sehr genau die virtuellen ortsbezogenen Informationen wiedergegeben werden, die dem betrachteten Teil 26 des Raumbereichs 14 zugeordnet sind.

Der Betrachter 20 blickt hier über die Anzeigevorrichtung 28 hinweg auf den betrachteten Teil 26 des Raumbereichs 14. Dies erfolgt in einer Sichtrichtung 30 von dem Kopf 24 des Betrachters 20 zu dem betrachteten Teil 26 des Raumbereichs 14 an der Wand 18.

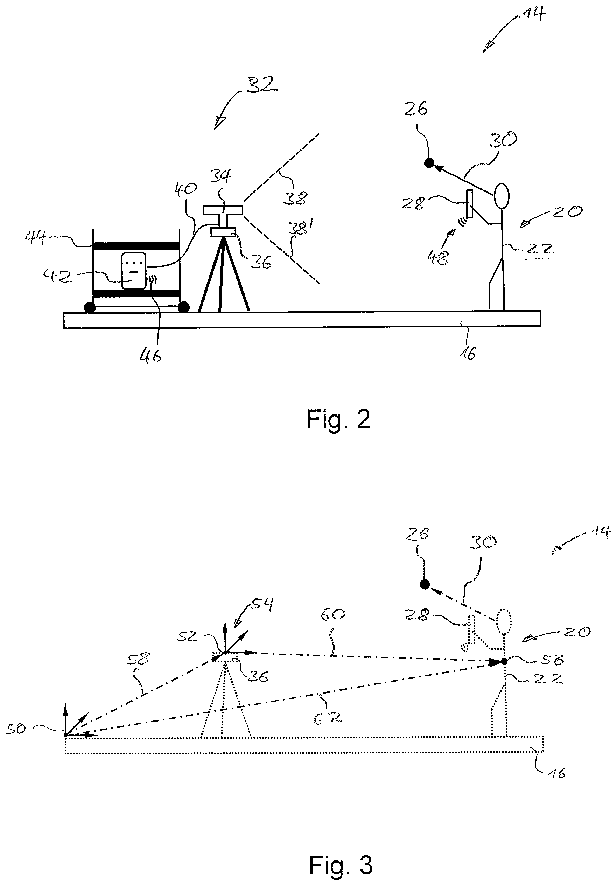

Fig. 2 zeigt den Beobachter 20 in schematischer Form zusammen mit einer Vorrichtung 32 zum Ermitteln und Wiedergeben der virtuellen ortsbezogenen Informationen. Die Vorrichtung 32 weist zusätzlich zu der Anzeigevorrichtung 28 eine Sensorvorrichtung 34 auf. Die Sensorvorrichtung 34 ist auf einem Dreibein 36 angeordnet. Hieraus ergibt sich eine ortsfeste Anordnung der Sensorvorrichtung 34 bezüglich des Raumbereichs 14. Zudem ist die Sensorvorrichtung 34 betrachterunabhängig in dem Raumbereich 14 angeordnet. Die Sensorvorrichtung 34 weist als Sensor eine 3D-Kamera auf. Die 3D-Kamera besteht aus einer Digitalkamera sowie einem Infrarot-Tiefenmesssensor. Der Vollständigkeit halber wird hier darauf hingewiesen, dass die Sensorvorrichtung nicht auf die Verwendung einer 3D-Kamera und einem Infrarot-Tiefenmesssensor beschränkt ist. Die einzelnen Sensoren der Sensorvorrichtung 34 sind aus Gründen der Übersichtlichkeit hier nicht dargestellt.

Die Sensorvorrichtung 34 erfasst den Betrachter 20 innerhalb eines Sensorbereichs, der hier durch Geraden 38 und 38' schematisch dargestellt wird. Der dargestellte Sensorbereich erfasst den Betrachter 20 im Gesamten. Denkbar sind auch Sensorbereiche, die lediglich Teile des Körpers 22 des Betrachters 20 erfassen.

Die von der Sensorvorrichtung 34 in dem Sensorbereich erfassten Informationen werden in dreidimensionale Messdaten umgewandelt, die über eine Leitung 40 an einen Computer 42 gesendet werden. Alternativ ist es denkbar, dass die Leitung 40 als drahtlose Funkstrecke ausgebildet ist. Der Computer 42 ist ebenfalls ein Teil der Vorrichtung 32. Er ist aus Gründen der Portabilität auf einem Wagen 44 angeordnet. Die dreidimensionalen Messdaten werden von dem Computer 42 empfangen, der dann die Sichtrichtung 30 des Betrachters 20 ermittelt.

Der Computer 42 weist weiter eine Sende- und Empfangsvorrichtung 46 auf. Über die Sende- und Empfangsvorrichtung 46 sendet der Computer 42 die Sichtrichtung 30 anschließend an die Anzeigevorrichtung 28. Die Anzeigevorrichtung 28 weist hierzu ebenfalls eine Sende- und Empfangsvorrichtung 48 auf. Die Sende- und Empfangsvorrichtungen 46 und 48 sind bevorzugt als drahtlose Sende- und Empfangsvorrichtungen 46 und 48 ausgebildet. Somit hat der Betrachter 20 eine größtmögliche Bewegungsfreiheit innerhalb des Raumbereichs 14.

In Abhängigkeit der Sichtrichtung 30 ermittelt die Anzeigevorrichtung 28, welcher Teil 26 des Raumbereichs 14 von dem Betrachter 20 aktuell betrachtet wird. Hierzu weist die Anzeigevorrichtung 28 eine nicht dargestellte Berechnungseinheit auf. Auf Basis dieser Information werden die virtuellen ortsbezogenen Informationen aus einer entsprechenden Datenbank ausgewählt. Anschließend werden die virtuellen ortsbezogenen Informationen von der Anzeigevorrichtung 28 wiedergegeben. In diesem Ausführungsbeispiel bilden der Computer 42 und die Berechnungseinheit der Anzeigevorrichtung 28 gemeinsam eine Steuer- und Auswerteeinheit.

Fig. 3 zeigt den Raumbereich 14, den Betrachter 20 sowie einen Teil der Vorrichtung 32 in gestrichelter Darstellung. Dem Raumbereich 14 ist ein Koordinatensystem 50 zugeordnet. Dieses Koordinatensystem 50 entspricht vorzugsweise einem virtuellen Koordinatensystem für die virtuellen ortsbezogenen Informationen innerhalb der Datenbank. Dieses kann beispielsweise ein Bezugssystem für CAD-Daten sein.

Das Dreibein 36 dient als Referenz für die Sensorvorrichtung 34 im Raumbereich 14. Die Sensorposition 52 wird daher als Raumpunkt auf dem Dreibein 36 angenommen. Die Sensorposition 52 ist zudem ein Ursprung eines Koordinatensystems 54 für die Sensorvorrichtung 34. Die Lage des Koordinatensystems 54 bestimmt eine Sensorausrichtung der Sensorvorrichtung 34 innerhalb des Raumbereichs 14 und relativ zu dem Koordinatensystem 50. Dem Betrachter 20 ist eine Raumposition 56 zugeordnet. Diese Raumposition 56 ist hier ebenfalls als Raumpunkt ausgebildet, der sich innerhalb des Körpers 22 des Betrachters 20 befindet.

Ausgehend von dem Koordinatensystem 50 zu der Sensorposition 52 ergibt sich ein Raum-Sensor-Vektor 58. Dieser Vektor 58 ist der Sensorvorrichtung 34 und/oder der Steuer- und Auswerteeinheit bekannt, beispielsweise aus einem Kalibriervorgang. Die Sensorvorrichtung 34 ermittelt die Messdaten in dem Koordinatensystem 54. Aufgrund des bekannten Raum-Sensor-Vektors 58 können diese Messdaten in das Koordinatensystem 50 des Raumbereichs 14 transformiert werden. Hierbei sind die Lage und Ausrichtung des Koordinatensystems 54 bezüglich des Koordinatensystems 50 zu berücksichtigen. Ausgehend von der Sensorposition 52 erstreckt sich zudem ein Sensor-Betrachter-Vektor 60 zu der Raumposition 56. Dieser Sensor-Betrachter-Vektor 60 wird in Abhängigkeit der Messdaten ermittelt. Durch Transformation des Sensor-Betrachter-Vektors 60 kann die Raumposition 56 in dem Koordinatensystem 50 dargestellt werden. Hieraus ergibt sich ein Raum-Betrachter-Vektor 62, der die Raumposition 56 des Betrachters 20 relativ zu dem Raum 14 definiert.

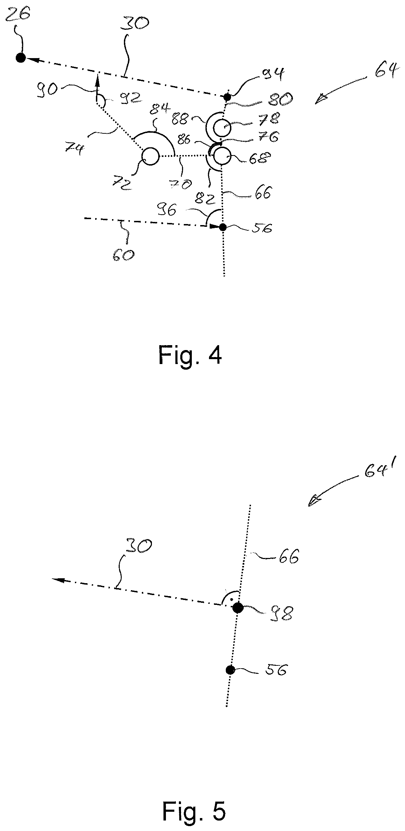

Fig. 4 zeigt ein Skelettmodell 64 des Betrachters 20. Das Skelettmodell 64 repräsentiert die Körperhaltung des Betrachters 20 innerhalb des Computers 42. In Abhängigkeit des Skelettmodells 64 kann die Körperhaltung des Betrachters 20 analysiert und es kann somit die Sichtrichtung 30 ermittelt werden. Das Skelettmodell 64 ist ein dreidimensionales Skelettmodell. Aus Gründen der Übersichtlichkeit ist es hier zweidimensional dargestellt. Das Skelettmodell 64 weist vereinfachte Repräsentationen der einzelnen Körperteile des Betrachters 20 in Form von Geraden auf. Diese Repräsentationen werden mittels Gelenkpunkten miteinander verbunden. Zudem werden Winkel zwischen den einzelnen Körperteilen definiert, um die Körperhaltung vollständig und bestimmt repräsentieren zu können.

Das hier dargestellte Skelettmodell 64 entspricht einem Teil des Körpers 22 des Betrachters 20. Es repräsentiert einen Brustbereich, einen Armbereich einen Halsbereich und einen Kopfbereich des Betrachters 20. Zur Repräsentation dieser Bereiche des Körpers 22 weist das Skelettmodell 64 entsprechend ein Brustteil 66 auf. Dieses ist über ein Schultergelenk 68 mit einem Oberarmteil 70 verbunden. Das Oberarmteil 70 ist weiter über ein Ellenbogengelenk 72 mit einem Unterarmteil 74 verbunden. Ausgehend von dem Schultergelenk 68 erstreckt sich ein Halsteil 76 zu einem Gelenk 78. Das Gelenk 78 repräsentiert eine Schwenkbarkeit des Kopfs 24 des Betrachters 20. Ausgehend von dem Gelenk 78 erstreckt sich ein Kopfteil 80.

Jedem der Gelenke 68, 72 und 78 sind entsprechende Winkel zugeordnet, die die räumliche Ausrichtung der einzelnen Körperteile definieren. Ein Winkel 82 beschreibt eine relative räumliche Ausrichtung von dem Brustteil 66 zu dem Oberarmteil 70. Ein Winkel 84 beschreibt eine relative räumliche Ausrichtung von dem Oberarmteil 70 zu dem Unterarmteil 74. Ein weiterer Winkel 86 beschreibt eine relative räumliche Ausrichtung von dem Oberarmteil 70 zu dem Halsteil 76. Ein weiterer Winkel 88 beschreibt eine relative räumliche Ausrichtung von dem Halsteil 76 zu dem Kopfteil 80.

In Abhängigkeit dieser Informationen kann die Körperhaltung des Betrachters 20 im Raum sehr genau beschrieben werden. Zur Ermittlung der Sichtrichtung 30 wird hier zusätzlich angenommen, dass der Betrachter 20 über die Anzeigevorrichtung 28 drüber blickt. Daher wird zusätzlich ein Hindernis 90 vordefinierter Länge berücksichtigt, welches sich von einem Ende des Unterarmteils 74 unter einem Winkel 92 erstreckt.

Die Sichtrichtung 30 wird dadurch ermittelt, dass ein Ursprungspunkt 94 am Kopfteil 80 definiert wird, aus dem sich die Sichtrichtung 30 erstreckt. Ausgehend von dem Ursprungspunkt 94 verläuft die Sichtrichtung 30 dann über das Hindernis 90 hinweg. Um die Körperhaltung in dem Koordinatensystem 50 des Raumbereichs 14 zu beschreiben, wird zudem die Raumposition 56 sowie ein Winkel 96 zwischen dem Sensor-Betrachter-Vektor 60 und dem Brustteil 66 berücksichtigt. Insgesamt ergibt sich daraus die Sichtrichtung 30 innerhalb des Koordinatensystems 50.

In Abhängigkeit der Sichtrichtung 30 und der gespeicherten virtuellen Informationen über den Raumbereich 14 wird dann ermittelt, auf welchen Teil 26 des Raumbereichs 14 der Betrachter 20 aktuell blickt.

Zudem ist eine alternative, vereinfachte Möglichkeit zur Ermittlung der Sichtrichtung denkbar. Diese Alternative ist nicht explizit in den Figuren dargestellt. Zur Ermittlung der Sichtrichtung wird ein vereinfachtes Skelettmodell des Betrachters ermittelt. Das Skelettmodell weist dann lediglich einen Oberarmteil 70 sowie ein Unterarmteil 74 auf, wie sie in Fig.4 gezeigt sind. In dieser Ausführungsform wird die Sichtrichtung unter der Annahme ermittelt, dass sich die Sichtrichtung von dem Ellenbogengelenk 72 durch den Unterarmteil 74 hindurch erstreckt. Somit kann die Sichtrichtung entsprechend wie die Sichtrichtung 30 aus Fig. 4 ermittelt werden, wobei die Sichtrichtung einer Zeigerichtung des Betrachters entspricht. Der Betrachter kann dann den erfassten Arm ähnlich einem Zeigestock zur Veränderung der Sichtrichtung einsetzen. Vorteil hierbei ist, dass eine sehr intuitive Steuerung der Sichtrichtung möglich ist, wobei gleichzeitig eine sehr einfache Ermittlung der Sichtrichtung ermöglicht wird. Ein weiterer Vorteil ist, dass die Verwendung des Unterarmteils zur direkten Bestimmung der Sichtrichtung die Stabilität beim Ermitteln der Sichtrichtung erhöht.

Fig. 5 zeigt eine weitere, vereinfachte Möglichkeit zur Ermittlung der Sichtrichtung 30. Hierbei wird die Sichtrichtung 30 unter der Annahme ermittelt, dass sie als Normale zu einem Brustbereich des Betrachters 20 verläuft. Unter dieser Annahme ist es ausreichend, wenn das Brustteil 66 in seiner räumlichen Ausrichtung als Skelettmodell 64' erfasst wird. Ausgehend von einem vordefinierten Ursprungspunkt 98 kann dann die Sichtrichtung 30 als Normale zu dem Brustteil 66 ermittelt werden. Das Brustteil 66 wird bevorzugt als Fläche repräsentiert, um die Ausrichtung der Sichtrichtung 30 im Raum einfach und eindeutig ermitteln zu können.

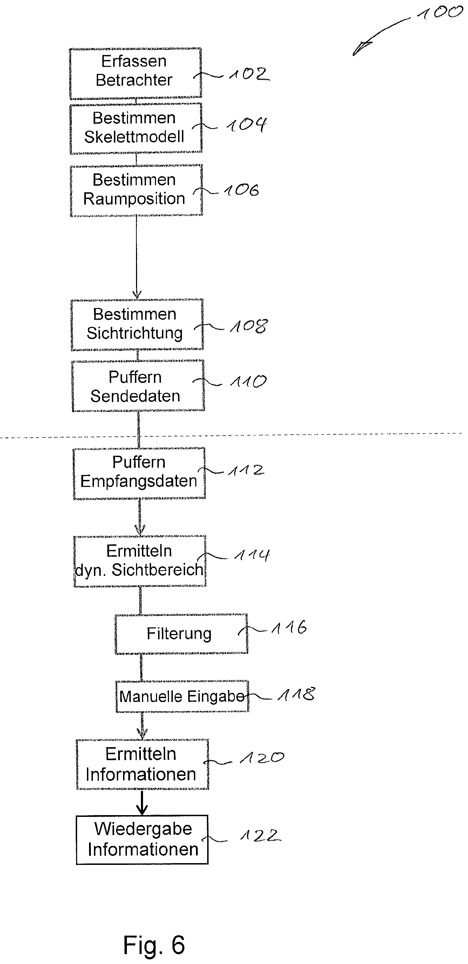

Fig. 6 zeigt ein Flussdiagramm 100 eines Ausführungsbeispiels des erfindungsgemäßen Verfahrens. In einem ersten Schritt 102 wird der Betrachter 20 von der Sensorvorrichtung 34 erfasst. Dies erfolgt zum einen mittels der Digitalkamera, die Farbbilder als Messdaten aufnimmt. Zum anderen werden mittels des Infrarot-Tiefensensors Tiefeninformationen als Messdaten ermittelt. Zusammen ergeben sich aus den Messdaten der Digitalkamera und des Infrarot-Tiefensensors dreidimensionale Messdaten, die an den Computer 42 gesendet werden.

In einem weiteren Schritt 104 bildet der Computer 42 das Skelettmodell 64 des Betrachters in Abhängigkeit der dreidimensionalen Messdaten.

Weiter wird in einem Schritt 106 die Raumposition 56 des Betrachters in Abhängigkeit der dreidimensionalen Messdaten bestimmt.

In einem Schritt 108 wird die Sichtrichtung 30, wie vorstehend beschrieben, in Abhängigkeit des Skelettmodells 64, und damit in Abhängigkeit der Körperhaltung des Betrachters 20, und in Abhängigkeit der Raumposition 56 ermittelt.

Die so ermittelte Sichtrichtung 30 wird dann in einem Schritt 110 als Sendedaten in einem Sendespeicher im Computer 42 zwischengespeichert. Die Sendedaten werden an einen entsprechenden Empfangsspeicher in der Anzeigevorrichtung 28 übermittelt, der die Sendedaten als Empfangsdaten zwischenspeichert. Die Zwischenspeicherungen der Sendedaten in Schritt 110 und der Empfangsdaten in Schritt 112 dienen dazu, dass die Wiedergabe der virtuellen ortsbezogenen Informationen sicher und flüssig erfolgt, auch wenn unbrauchbare oder unvorhergesehene Messdaten von der Sensorvorrichtung 34 ermittelt werden, oder eine Kommunikationsverbindung zwischen dem Computer 42 und der Anzeigevorrichtung 28 kurzzeitig nicht verfügbar ist.

In einem weiteren Schritt 114 wird ein dynamischer Sichtbereich bestimmt. Dies erfolgt mit einer Sliding-Window-Frame-Berechnung. Hierbei wird eine bisherige Bewegung des Sichtvektors 30 berücksichtigt. Durch das Bestimmen des dynamischen Sichtbereichs wird vorbereitet, dass die virtuellen ortsbezogenen Informationen auf der Anzeigevorrichtung als gleitende Ansicht dargestellt werden. In anderen Worten, dass die virtuellen Informationen mit der Sichtrichtung 30 übergangslos mitwandern. Hieraus ergibt sich eine dynamische Darstellung der virtuellen ortsbezogenen Informationen, wobei nicht einzelne Bilder unabhängig von der Bewegung auf der Anzeigevorrichtung 28 dargestellt werden, sondern fließende Übergänge über die angezeigten virtuellen ortsbezogenen Informationen ermöglicht werden.

Vorzugsweise wird die Bewegung der Sichtrichtung in Schritt 114 zusätzlich geglättet. Dies kann beispielsweise durch eine Fast-Fourier-Transformation der Sichtrichtung 30 in Verbindung mit einer Tiefpassfilterung und einer inversen Fast-Fourier-Transformation erfolgen. Bildlich gesprochen ergibt sich hieraus, dass die von der Anzeigevorrichtung 28 wiedergegebenen virtuellen ortsbezogenen Informationen besonders weich und synchron zu den Bewegungen der Sichtrichtung 30 gleiten.

In einem weiteren Schritt 116 erfolgt eine Filterung der in Schritt 114 gewonnenen Daten, um einen Rechenaufwand zu minimieren. Hierbei wird eine Frame-Rate zur Wiedergabe der virtuellen ortsbezogenen Informationen auf der Anzeigevorrichtung 28 reduziert. Dies erfolgt vorzugsweise auf drei Frames pro Sekunde.

In einem weiteren Schritt 118 besteht zusätzlich die Möglichkeit, dass der Betrachter 20 die aktuell wiedergegebenen virtuellen ortsbezogenen Informationen manuell modifiziert. Zum einen kann der Betrachter 20 eine aktuell vorliegende Sichtrichtung 30 fixieren. Somit kann er die aktuell dargestellten virtuellen ortsbezogenen Informationen unabhängig von seinen Bewegungen betrachten. Zusätzlich ist es vorgesehen, dass durch manuelle Eingaben des Betrachters 20 in seiner Anzeigevorrichtung 28 die Sichtrichtung 30 modifiziert werden kann. Hierfür weist die Anzeigevorrichtung 28 Eingabemittel wie beispielsweise ein Touchscreen auf, mit dem der Betrachter 20 die Sichtrichtung 30 manuell verändern kann.

In einem weiteren Schritt 120 werden in Abhängigkeit der Sichtrichtung 30 die virtuellen ortsbezogenen Informationen ermittelt, welche der jeweiligen Sichtrichtung 30 entsprechen.

Schließlich erfolgt in einem Schritt 122 die Wiedergabe der so ermittelten virtuellen ortsbezogenen Informationen.

Die vorliegende Erfindung ermöglicht somit eine sehr robuste, schnelle und genaue Ermittlung und Wiedergabe von virtuellen ortsbezogenen Informationen, wobei gleichzeitig eine einfache Bedienung durch den Betrachter gewährleistet ist. Weiter ermöglicht die Erfindung, dass der Betrachter ohne spezielle Markierungen hierfür an seinem Körper auskommt. Insgesamt gibt die Erfindung dem Betrachter eine größtmögliche Freiheit zur Betrachtung seiner Umgebung bei gleichzeitiger Robustheit, Schnelligkeit und Genauigkeit der ermittelten und wiedergegebenen ortsbezogenen Informationen.

In weiteren, hier nicht dargestellten, Ausführungsbeispielen, kann die Steuer- und Auswerteeinheit anderweitig ausgebildet sein. Beispielsweise kann der Computer 42 auch die virtuellen ortsbezogenen Informationen ermitteln, wobei dann der Anzeigevorrichtung beispielsweise nur Grafikinformationen übermittelt werden, ähnlich einem Terminalsystem. Auch ist es denkbar, dass die Anzeigevorrichtung 28 die gesamte Steuer- und Auswerteeinheit bereitstellt, wobei dann ein externer Computer 42 entfallen würde. Denkbar ist weiter eine teilweise oder vollständige Integration der Steuer- und Auswerteeinheit in die Sensorvorrichtung 34. Diese Ausführungsbeispiele führen insbesondere zu einer sehr kompakten Ausgestaltung der Vorrichtung 32. Gleichzeitig besteht die Möglichkeit, dass bereits verfügbare Komponenten, wie bereits bestehende Sensorvorrichtungen und/oder Anzeigevorrichtungen, auf wirtschaftliche Weise zur Herstellung der Vorrichtung verwendet werden können.

Die Erfindung wurde mit Bezug zu den Figuren exemplarisch dargestellt und im Detail beschrieben. Diese Darstellung und Beschreibung sollen als illustrativ oder beispielhaft nicht jedoch als einschränkend verstanden werden. Es versteht sich, dass die vorstehend genannten Merkmale nicht nur in der jeweils angegebenen Kombination, sondern auch in anderen Kombinationen oder in Alleinstellung verwendbar sind, ohne den Rahmen der vorliegenden Erfindung zu verlassen.

In den Ansprüchen schließt der Begriff "mit" keine weiteren Elemente oder Schritte aus. Weiter schließt die Verwendung unbestimmter Artikel nicht aus, dass eine Vielzahl von gleichartigen Elementen vorhanden ist oder eine Vielzahl von gleichartigen Schritten ausgeführt wird. Ein einzelnes Element oder eine andere Einheit kann die Funktionen mehrerer Elemente aus den Ansprüchen erfüllen. Die bloße Tatsache, dass bestimmte Maßnahmen in unterschiedlichen abhängigen Ansprüchen beschrieben werden, schließt nicht aus, dass eine Kombination dieser Maßnahmen gemeinsam genutzt werden kann.

Ein Computerprogramm kann auf einem geeigneten nicht flüchtigen Medium gespeichert oder bereitgestellt werden, wie beispielsweise einem optischen Speichermedium oder einem Solid-State-Medium zusammen mit oder als Teil von weiterer Hardware. Das Computerprogramm kann aber auch auf andere Weise bereitgestellt werden, beispielsweise über das Internet oder andere drahtgebundene oder drahtlose Telekommunikationssysteme.

Bezugszeichen in den Ansprüchen beschränken nicht deren Umfang.

## Patentansprüche

1. Verfahren zum Ermitteln und Wiedergeben virtueller ortsbezogener Informationen für einen Raumbereich (14), mit den Schritten:
- Ermitteln von Messdaten von mindestens einem Teil eines Körpers (22) eines Betrachters (20) in dem Raumbereich (14) mittels einer Sensorvorrichtung (34), die betrachterunabhängig und/oder ortsfest in dem Raumbereich (14) angeordnet wird,
- Bildung eines Skelettmodell (64, 64') in Abhängigkeit der Messdaten,
- Ermitteln einer Körperhaltung des Betrachters (20) in Abhängigkeit der Messdaten mittels des Skelettmodells (64, 64'),
- Bestimmen einer Raumposition (56) des Betrachters (20) relativ zu dem Raumbereich (14),
- Ermitteln einer Sichtrichtung (30) des Betrachters (20) relativ zu dem Raumbereich (14) in Abhängigkeit der Raumposition (56) und der Körperhaltung,
- Ermitteln der virtuellen ortsbezogenen Informationen in Abhängigkeit der Sichtrichtung (30), und
- Wiedergeben der virtuellen ortsbezogenen Informationen mittels einer Anzeigevorrichtung (28).

2. Verfahren nach Anspruch 1, **dadurch gekennzeichnet, dass** als Messdaten dreidimensionale Messdaten ermittelt werden.

3. Verfahren nach einem der Ansprüche 1 oder 2, **dadurch gekennzeichnet, dass** die Sensorvorrichtung (34) automatisch eine Sensorposition (52) und/oder eine Sensorausrichtung relativ zu dem Raumbereich (14) ermittelt.

4. Verfahren nach einem der Ansprüche 1 bis 3, **dadurch gekennzeichnet, dass** eine Vielzahl von Sensorvorrichtungen (34) eingesetzt wird.

5. Verfahren nach einem der Ansprüche 1 bis 4, **dadurch gekennzeichnet, dass** als der mindestens eine Teil des Körpers (22) des Betrachters (20) mindestens ein Armbereich und/oder ein Brustbereich des Betrachters (20) verwendet werden.

6. Verfahren nach einem der Ansprüche 1 bis 5, **dadurch gekennzeichnet, dass** eine Bewegung der Sichtrichtung (30) erfasst und die Sichtrichtung (30) in Abhängigkeit der Bewegung ermittelt wird.

7. Verfahren nach einem der Ansprüche 1 bis 6, **dadurch gekennzeichnet, dass** als virtuelle ortsbezogene Informationen CAD-Daten verwendet werden.

8. Verfahren nach einem der Ansprüche 1 bis 7, **dadurch gekennzeichnet, dass** als Anzeigevorrichtung (28) ein Tablet-Computer (28) verwendet wird.

9. Verfahren nach einem der Ansprüche 1 bis 8, **dadurch gekennzeichnet, dass** zusätzlich reale ortsbezogene Informationen auf der Anzeigevorrichtung (28) wiedergegeben werden.

10. Vorrichtung (32) zum Ermitteln und Wiedergeben virtueller ortsbezogener Informationen für einen Raumbereich (14), mit
einer Sensorvorrichtung (34), die betrachterunabhängig und/oder ortsfest in dem Raumbereich (14) angeordnet wird, zur Ermittlung von Messdaten von mindestens einem Teil eines Körpers (22) eines Betrachters (20) in dem Raumbereich (14),
einer Steuer- und Auswerteeinheit (42) zur Bildung eines Skelettmodell (64, 64') in Abhängigkeit der Messdaten, zur Ermittlung einer Körperhaltung des Betrachters (20) in Abhängigkeit der Messdaten mittels des Skelettmodells (64, 64'), zur Bestimmung einer Raumposition (56) des Betrachters (20) relativ zu dem Raumbereich (14), zur Ermittlung einer Sichtrichtung (30) des Betrachters (20) relativ zu dem Raumbereich (14) in Abhängigkeit der Raumposition (56) und der Körperhaltung und zur Ermittlung der virtuellen ortsbezogenen Informationen in Abhängigkeit der Sichtrichtung (30), und
einer Anzeigevorrichtung (28) zum Wiedergeben der virtuellen ortsbezogenen Informationen.

11. Steuer- und Auswerteeinheit (42) für eine Vorrichtung (32) zum Ermitteln und Wiedergeben virtueller ortsbezogener Informationen für einen Raumbereich (14), mit
Mitteln zum Empfangen von Messdaten einer betrachterunabhängig und/oder ortsfest in dem Raumbereich (14) angeordneten Sensorvorrichtung (34), die die Messdaten von mindestens einem Teil eines Körpers (22) eines Betrachters (20) in dem Raumbereich (14) ermittelt,
Mitteln zur Bildung eines Skelettmodell (64, 64') in Abhängigkeit der Messdaten,
Mitteln zur Ermittlung einer Körperhaltung des Betrachters (20) in Abhängigkeit der Messdaten mittels des Skelettmodells (64, 64'),
Mitteln zur Bestimmung einer Raumposition (56) des Betrachters (20) relativ zu dem Raumbereich (14),
Mitteln zur Ermittlung einer Sichtrichtung (30) des Betrachters (20) relativ zu dem Raumbereich (14) in Abhängigkeit der Raumposition (56) und der Körperhaltung,
Mitteln zur Ermittlung der virtuellen ortsbezogenen Informationen in Abhängigkeit der Sichtrichtung (30), und
Mitteln zur Bereitstellung der virtuellen ortsbezogene Informationen zum Wiedergeben der virtuellen ortsbezogene Informationen.

12. Verfahren zum Betreiben einer Steuer- und Auswerteeinheit (42) für eine Vorrichtung (32) zum Ermitteln und Wiedergeben virtueller ortsbezogener Informationen für einen Raumbereich (14), mit den Schritten:
- Empfangen von Messdaten einer betrachterunabhängig und/oder ortsfest in dem Raumbereich (14) angeordneten Sensorvorrichtung (34), die die Messdaten von mindestens einem Teil eines Körpers (22) eines Betrachters (20) in dem Raumbereich (14) ermittelt,
- Bildung eines Skelettmodell (64, 64') in Abhängigkeit der Messdaten,
- Ermitteln einer Körperhaltung des Betrachters (20) in Abhängigkeit der Messdaten mittels des Skelettmodells (64, 64'),
- Bestimmen einer Raumposition (56) des Betrachters (20) relativ zu dem Raumbereich (14),
- Ermitteln einer Sichtrichtung (30) des Betrachters (20) relativ zu dem Raumbereich (14) in Abhängigkeit der Raumposition (56) und der Körperhaltung,
- Ermitteln der virtuellen ortsbezogenen Informationen in Abhängigkeit der Sichtrichtung (30), und
- Bereitstellen der virtuellen ortsbezogenen Informationen zum Wiedergeben der virtuellen ortsbezogenen Informationen.

13. Computerprogramm mit Programmcodemitteln zum Durchführen der Schritte eines Verfahrens nach Anspruch 12, wenn das Computerprogramm auf einem Computer (42) ausgeführt wird.

## Claims

1. A method for determining and reproducing virtual, position-related information for a region of space (14), comprising the steps:
- detecting measurement data from at least one part of a body (22) of a viewer (20) in the region of space (14) using a sensor device (34), which is arranged independently of the vier and/or in a fixed position in the region of space (14),
- forming a skeleton model (64, 64') on the basis of the measurement data,
- determining a posture of the viewer (20) on the basis of the measurement data using the skeleton model (64, 64'),
- identifying a spatial position (56) of the viewer (20) relative to the region of space (14),
- determining a viewing direction (30) of the viewer (20) relative to the region of space (14) on the basis of the spatial position (56) and the posture,
- determining the virtual, position-related information on the basis of the viewing direction (30), and
- using a display device (28) to reproduce the virtual, position-related information.

2. The method as claimed in claim 1, **characterized in that** three-dimensional measurement data is detected as the measurement data.

3. The method as claimed in any of claims 1 or 2, **characterized in that** the sensor device (34) automatically determines a sensor position (52) and/or a sensor orientation relative to the region of space (14).

4. The method as claimed in any of claims 1 to 3, **characterized in that** a multiplicity of sensor devices (34) are used.

5. The method as claimed in any of claims 1 to 4, **characterized in that** at least an arm region and/or a chest region of the viewer (20) is used as the at least one part of the body (22) of the viewer (20).

6. The method as claimed in any of claims 1 to 5, **characterized in that** a movement of the viewing direction (30) is detected, and the viewing direction (30) is determined on the basis of the movement.

7. The method as claimed in any of claims 1 to 6, **characterized in that** CAD data is used as the virtual, position-related information.

8. The method as claimed in any of claims 1 to 7, **characterized in that** a tablet computer (28) is used as the display device (28).

9. The method as claimed in any of claims 1 to 8, **characterized in that** additional real, position-related information is reproduced on the display device (28).

10. A device (32) for determining and reproducing virtual, position-related information for a region of space (14), comprising
a sensor device (34), which is arranged independently of the vier and/or in a fixed position in the region of space (14), for detecting measurement data from at least one part of a body (22) of a viewer (20) in the region of space (14),
a control and analysis unit (42) for forming a skeleton model (64, 64') on the basis of the measurement data, for determining a posture of the viewer (20) on the basis of the measurement data using the skeleton model (64, 64'), for identifying a spatial position (56) of the viewer (20) relative to the region of space (14), for determining a viewing direction (30) of the viewer (20) relative to the region of space (14) on the basis of the spatial position (56) and the posture, and for determining the virtual, position-related information on the basis of the viewing direction (30),
and a display device (28) for reproducing the virtual, position-related information.

11. A control and analysis unit (42) for a device (32) for determining and reproducing virtual, position-related information for a region of space (14), comprising
means for receiving measurement data from a sensor device (34), which is arranged independently of the vier and/or in a fixed position in the region of space (14) and which detects measurement data from at least one part of a body (22) of a viewer (20) in the region of space (14),
means for determining a posture of the viewer (20) on the basis of the measurement data,
means for forming a skeleton model (64, 64') on the basis of the measurement data,
means for identifying a spatial position (56) of the viewer (20) relative to the region of space (14) using the skeleton model (64, 64'),
means for determining a viewing direction (30) of the viewer (20) relative to the region of space (14) on the basis of the spatial position (56) and the posture,
means for determining the virtual, position-related information on the basis of the viewing direction (30), and
means for providing the virtual, position-related information for reproducing the virtual, position-related information.

12. A method for operating a control and analysis unit (42) for a device (32) for determining and reproducing virtual, position-related information for a region of space (14), comprising the steps:
- receiving measurement data from a sensor device (34), which is arranged independently of the vier and/or in a fixed position in the region of space (14) and which detects measurement data from at least one part of a body (22) of a viewer (20) in the region of space (14),
- forming a skeleton model (64, 64') on the basis of the measurement data,
- determining a posture of the viewer (20) on the basis of the measurement data using the skeleton model (64, 64'),
- identifying a spatial position (56) of the viewer (20) relative to the region of space (14),
- determining a viewing direction (30) of the viewer (20) relative to the region of space (14) on the basis of the spatial position (56) and the posture,
- determining the virtual, position-related information on the basis of the viewing direction (30), and
- providing the virtual, position-related information for reproducing the virtual, position-related information.

13. A computer program comprising program code means for implementing the steps of a method as claimed in claim 12 when the computer program is executed on a computer (42).

## Revendications

1. Procédé permettant de déterminer et de reproduire des informations de localisation virtuelles concernant une zone spatiale (14), comprenant les étapes consistant à :
- déterminer des données de mesure d'au moins une partie d'un corps (22) d'un observateur (20) dans la zone spatiale (14) au moyen d'un dispositif capteur (34) qui est disposé dans la zone spatiale (14) indépendamment de l'observateur et/ou de manière fixe,
- créer un modèle de squelette (64, 64') en fonction des données de mesure,
- déterminer une posture corporelle de l'observateur (20) en fonction des données de mesure au moyen du modèle de squelette (64, 64'),
- déterminer une position spatiale (56) de l'observateur (20) par rapport à la zone spatiale (14),
- déterminer une direction du regard (30) de l'observateur (20) par rapport à la zone spatiale (14) en fonction de la position spatiale (56) et de la posture corporelle,
- déterminer les informations de localisation virtuelles en fonction de la direction du regard (30), et
- reproduire les informations de localisation virtuelles au moyen d'un dispositif d'affichage (28).

2. Procédé selon la revendication 1, **caractérisé en ce que** des données de mesure tridimensionnelles sont déterminées en tant que données de mesure.

3. Procédé selon l'une des revendications 1 ou 2, **caractérisé en ce que** le dispositif capteur (34) détermine automatiquement une position de capteur (52) et/ou une orientation de capteur par rapport à la zone spatiale (14).

4. Procédé selon l'une des revendications 1 à 3, **caractérisé en ce qu'**une pluralité de dispositifs capteurs (34) sont utilisés.

5. Procédé selon l'une des revendications 1 à 4, **caractérisé en ce qu'**au moins une zone de bras et/ou une zone de poitrine de l'observateur (20) sont utilisées en tant qu'au moins une partie du corps (22) de l'observateur (20).

6. Procédé selon l'une des revendications 1 à 5, **caractérisé en ce qu'**un mouvement de la direction du regard (30) est détecté et **en ce que** la direction du regard (30) est déterminée en fonction dudit mouvement.

7. Procédé selon l'une des revendications 1 à 6, **caractérisé en ce que** des données de CAO sont utilisées en tant qu'informations de localisation virtuelles.

8. Procédé selon l'une des revendications 1 à 7, **caractérisé en ce qu'**un ordinateur-tablette (28) est utilisé en tant que dispositif d'affichage (28).

9. Procédé selon l'une des revendications 1 à 8, **caractérisé en ce que** des informations de localisation réelles sont en outre reproduites sur le dispositif d'affichage (28).

10. Dispositif (32) permettant de déterminer et de reproduire des informations de localisation virtuelles concernant une zone spatiale (14), comportant
un dispositif capteur (34), qui est disposé dans la zone spatiale (14) indépendamment de l'observateur et/ou de manière fixe, permettant de déterminer des données de mesure pour au moins une partie d'un corps (22) d'un observateur (20) dans la zone spatiale (14), une unité de commande et d'évaluation (42) permettant former un modèle de squelette (64, 64') en fonction des données de mesure, de déterminer une posture corporelle de l'observateur (20) en fonction des données de mesure au moyen du modèle de squelette (64, 64'), de déterminer une position spatiale (56) de l'observateur (20) par rapport à la zone spatiale (14), de déterminer une direction du regard (30) de l'observateur (20) par rapport à la zone spatiale (14) en fonction de la position spatiale (56) et de la posture corporelle et de déterminer les informations de localisation virtuelles en fonction de la direction du regard (30), et
un dispositif d'affichage (28) permettant d'afficher les informations de localisation virtuelles.

11. Unité de commande et d'évaluation (42) destinée à un dispositif (32) permettant de déterminer et de reproduire des informations de localisation virtuelles concernant une zone spatiale (14), comportant
des moyens permettant de recevoir des données de mesure d'un dispositif capteur (34) qui est disposé indépendamment de l'observateur et/ou de manière fixe dans la zone spatiale (14) et qui détermine les données de mesure d'au moins une partie d'un corps (22) d'un observateur (20) dans la zone spatiale (14),
des moyens permettant de créer un modèle de squelette (64, 64') en fonction des données de mesure,
des moyens permettant de déterminer une posture corporelle de l'observateur (20) en fonction des données de mesure au moyen du modèle de squelette (64, 64'),
des moyens permettant de déterminer une position spatiale (56) de l'observateur (20) par rapport à la zone spatiale (14),
des moyens permettant de déterminer une direction du regard (30) de l'observateur (20) par rapport à la zone spatiale (14) en fonction de la position spatiale (56) et de la posture corporelle,
des moyens permettant de déterminer les informations de localisation virtuelles en fonction de la direction du regard (30), et
des moyens permettant de fournir les informations de localisation virtuelles afin de reproduire les informations de localisation virtuelles.

12. Procédé pour la mise en oeuvre d'une unité de commande et d'évaluation (42) destinée à un appareil (32) permettant de déterminer et de reproduire des informations de localisation virtuelles concernant une zone spatiale (14), comprenant les étapes consistant à :
- recevoir des données de mesure d'un dispositif capteur (34) qui est disposé dans la zone spatiale (14) indépendamment de l'observateur et/ou de manière fixe et qui détermine les données de mesure d'au moins une partie d'un corps (22) d'un observateur (20) dans la zone spatiale (14),
- créer un modèle de squelette (64, 64') en fonction des données de mesure,
- déterminer une posture corporelle de l'observateur (20) en fonction des données de mesure au moyen du modèle de squelette (64, 64'),
- déterminer une position spatiale (56) de l'observateur (20) par rapport à la zone spatiale (14),
- déterminer une direction du regard (30) de l'observateur (20) par rapport à la zone spatiale (14) en fonction de la position spatiale (56) et de la posture corporelle,
- déterminer les informations de localisation virtuelles en fonction de la direction du regard (30), et
- fournir les informations de localisation virtuelles afin de reproduire les informations de localisation virtuelles.

13. Programme d'ordinateur comportant des moyens à codes de programmes permettant de mettre en oeuvre les étapes d'un procédé selon la revendication 12 lorsque le programme d'ordinateur est exécuté sur un ordinateur (42).
